**Europäisches Patentamt**

**European Patent Office**

**Office européen des brevets**

(19)

(11) Veröffentlichungsnummer : **0 343 444 B1**

# EUROPÄISCHE PATENTSCHRIFT

(12)

(45) Veröffentlichungstag der Patentschrift :
**08.01.92 Patentblatt 92/02**

(51) Int. Cl.$^5$ : **A61K 7/48**

(21) Anmeldenummer : **89108536.7**

(22) Anmeldetag : **12.05.89**

(54) Kosmetisches Präparat auf Basis von Retinolpalmitat.

(30) Priorität : **25.05.88 DE 3817623**

(43) Veröffentlichungstag der Anmeldung :
**29.11.89 Patentblatt 89/48**

(45) Bekanntmachung des Hinweises auf die
Patenterteilung :
**08.01.92 Patentblatt 92/02**

(84) Benannte Vertragsstaaten :
**AT BE CH DE ES FR IT LI NL**

(56) Entgegenhaltungen :
**DE-A- 1 467 925**

(56) Entgegenhaltungen :
**GB-A- 906 000**
**G.A. NOWAK: "Die Kosmetischen Präparate:
Rezeptur, Rohstoffe, Wissenschaftliche
Grundlagen", Band 2, 1984, Seite 323, Augsburg, DE**

(73) Patentinhaber : **BAYER AG**
**W-5090 Leverkusen 1 Bayerwerk (DE)**

(72) Erfinder : **Siemer, Elisabeth, Dr.**
**Rochusweg 30**
**W-5300 Bonn (DE)**
Erfinder : **Voss, Eckart, Dr.**
**Johann-Janssen-Strasse 38**
**W-5090 Leverkusen 1 (DE)**

## Beschreibung

Die Erfindung betrifft ein kosmetisches Präparat zur lokalen Anwendung auf Basis von Retinolpalmitat (= Vitamin A-Palmitat).

Ein Mangel an Vitamin A äußert sich in trockener, rauher, verhornter Haut und Atrophie der Schweißdrüsen. Vitamin A (Retinol) wird deshalb seit langem in kosmetischen Mitteln zur Pflege bei trockener, schuppiger Altershaut empfohlen (siehe z.B. Reiss, F. und M. Campbell, Dermatologica 108, 121 (1961)).

Retinol übt neben seiner physiologischen Wirkung einen direkten pharmakologischen Effekt auf die Basalzellen der Haut aus. So wurde aufgrund wissenschaftlicher Untersuchungen nachgewiesen, daß bei topischer Anwendung von Retinolacetat die Mitoseaktivität der Haut um 30% gesteigert wurde. Ebenso konnte eine signifikante Verdickung der Epidermis nachgewiesen werden. Da auch die Enzymaktivität gesteigert wird, ist die Verwendung von Retinol besonders in Pflegepräparaten für die alternde Haut indiziert, da gerade zahlreiche Probleme der Altershaut durch die zu dünne Epidermis hervorgerufen werden.

Wegen der geringen Stabilität von Retinol werden in kosmetischen Präparaten meist Ester eingesetzt, vorzugsweise Retinolacetat und Retinolpalmitat. Retinolacetat penetriert leichter in die Haut als das Palmitat, ist allerdings in Emulsionen weniger stabil (Erlemann, G.A., Apotheker Journal, 7(5), 28-38 (1985)).

Nach JANISTYN sind die in der Fachliteratur angegebenen unterschiedlichen Ergebnisse der Wirkung von Vitaminen nach deren topischer Applikation dadurch zu erklären, daß die Aufnahme der Vitamine durch die Haut trägerabhängig ist (Janistyn, H., Handbuch der Kosmetika und Riechstoffe, III. Band : Die Körperpflegemittel, S. 614, Heidelberg 1973). Dies wird durch die Aussagen der Deutschen Patentschrift DE 2609575 C2 bestätigt, wonach die Mitosestimulierung der Haut durch Retinolacetat mittels Applikation in einer geeigneten Grundlage mit sehr viel niedrigeren Dosierungen erzielt werden kann als bei Anwendung des reinen Esters d.h. in Abwesenheit spezieller Zusatzstoffe.

Im Gegensatz zum Retinolacetat konnte in derselben Arbeit für Retinolpalmitat keine Wirkungssteigerung nachgewiesen werden. Eine Erhöhung der Epidermisverdickung war nach dieser Arbeit auch durch Steigerung der Retinolpalmitatkonzentration nicht möglich.

Dies bestätigt auch FLESCH, nach dem die zur Erhöhung der Epidermisdicke erforderlichen Retinolpalmitatdosen bei extrem hohen Werten von 50.000 bis 100.000 I.E./g liegen (FLESCH, P., J. Invest Dermatol. 19, 253 (1952)). Definition von I.E. (Internationale Einheit) : 1 I.E. = 0,3 µg Retinol (Vitamin A).

Überraschenderweise wurde jetzt gefunden, daß in einer die Verbindung 3,7,11,15-Tetramethyl-1,2,3-trihydroxyhexadecan enthaltenden Grundlage eine epidermale Mitosestimulierung und Epidermisverdickung schon mit sehr viel niedrigeren Retinolpalmitatdosen erzielt werden kann.

Die Erfindung betrifft somit ein kosmetisches Präparat in flüssiger, halbfester oder fester Form, vorzugsweise bestehend aus einer Öl-in-Wasser- oder Wasser-in-Öl-Emulsion oder einer wäßrigen oder öligen Lösung oder einem Gel oder einem Stift oder einem Aerosol als Grundlage, Retinolpalmitat in einer Menge von 1.000 bis 15.000 I.E./g und 0,01-1 Gew.-% 3,7,11,15-Tetramethyl-1,2,3-trihydroxy-hexadecan.

Das erfindungsgemäße Präparat enthält das Retinolpalmitat vorzugsweise in einer Konzentration von 2.000 bis 7.000 I.E./g, und 0,05 bis 0,5 Gew.-% 3,7,11,15-Tetramethyl-1,2,3-trihydroxy-hexadecan.

Das erfindungsgemäße kosmetische Mittel enthält neben der o.a. Wirkstoffkombination üblicherweise in kosmetischen Mitteln eingesetzte Träger- und Hilfsstoffe, insbesondere Antioxidantien wie alpha-Tocopherol, Butylhydroxyanisol, Butylhydroxytoluol u.a.

Zu den Träger- und Hilfsstoffen zählen desweiteren Lösungsmittel wie Wasser, Monoalkohole, niedrige Polyalkohole mit 1-6 Kohlenstoffatomen oder Mischungen davon, weiterhin Fettkörper, wie mineralische, tierische, oder pflanzliche Öle oder Wachse, Fettsäuren, Fettalkohole, Fettsäureester, Fettalkoholether, oxyethylierte Fettalkohole, Lanolin und — Derivate, Silikonöle.

Das erfindungsgemäße kosmetische Mittel kann auch Emulgatoren enthalten, wobei es sich um nichtionische, anionische, kationische oder amphotere Verbindungen handelt, z.B. Sterole, Polyol-Fettsäureester und -Fettalkoholether, Alkali- oder Triethanolaminsalze von Fettsäuren, Natriumcetylstearylsulfat, Tetraacylammoniumhalogenide, Phospholipide.

Ferner können Verdickungsmittel im erfindungsgemäßen Mittel eingesetzt sein. Dazu zählen Polyacrylsäurederivate, Cellulosederivate, Bentonite, Xanthanderivate, Alginate, Guarmehl und Johannisbrotmehl.

Das erfindungsgemäße Präparat kann weitere in kosmetischen Mitteln übliche Stoffe enthalten. Dazu zählen Feuchthaltemittel, UV-Filter, Farbstoffe, Puffersubstanzen, Konservierungsmittel und Parfümöle.

Als Feuchthaltemittel seien beispielhaft aufgeführt :

Niedrige Polyalkohole wie Glycerin, Propylenglykol, Butylenglykol, Sorbitol, des weiteren die 2-Pyrrolidon-5-Carbonsäure und ihr Natriumsalz, Milchsäure und ihre Salze, Harnstoff, Proteine und Proteinderivate wie Collagen, des weiteren Hyaluronsäure u.a.

Als UV-Filter kommen vorzugsweise in Frage : Benzylidenkampferverbindungen, p-Aminobenzoesäure sowie ihre Derivate, Anthranilate, Cinnamate, Salicylate, Benzoxazol-Derivate ; Benzophenonderivate, Dibenzoylmethanderivate, Benzotriazol-Derivate.

Insbesondere setzt man die folgenden Verbindungen als UV-Filter ein :

2-Ethoxyhexyl-p-(dimethylamino)-benzoat ;

2-Ethylhexyl-p-methoxycinnamat ;

3-Benzyliden-d,1-kampfer ;

3-(4'-Methylbenzyliden)-d,1-kampfer ;

Amyl-4-(dimethylamino)-benzoat ;

Homomenthylsalicylat ;

2-Hydroxy-4-methoxybenzophenon ;

N-(2-Ethylhexyl)-4-(3'-methylidenkampfer)-benzolsulfonamid ;

N-(2-Ethylhexyl)-3-benzyliden-10-kampfersulfonamid, gegebenenfalls zusammen mit einer der folgenden Verbindungen :

4-tert.-Butyl-4'methoxydibenzoylmethan ;

4-Isopropyldibenzoylmethan ;

α-(2-Oxo-3-bornyliden)-toluol-4-sulfonsäure und die Salze davon ;

α-(2-Oxo-3-bornyliden)-p-xylol-2-sulfonsäure ;

2-Hydroxy-4-methoxybenzophenon-5-sulfonsäure ;

4-[(2-Oxo-3-bornyliden)-methyl)-phenyltrimethylammoniummethylsulfat ;

1,4-Di-[sulfocamphorylmethyliden]-benzolsäure ;

2-Phenylbenzimidazol-5-sulfonsäure.

Als den erfindungsgemäßen kosmetischen Präparaten zuzusetzenden Farbstoffe seien beispielhaft aufgeführt :

Farbe L Rot 4, Farbe C Grün 11, Farbe L Blau 3, Sicomet Gelborange 85, Sicomet Patentblau, Sicopharm Gelb 10.

Als Konservierungsmittel kommen vorzugsweise in Frage :

2,4-Hexadiensäure (Sorbinsäure und ihre Salze)

4-Hydroxybenzoesäure, ihre Salze und Ester,

3-Acetyl-6-methyl-2,4(3H)-pyrandion (Dehydracetsäure) und seine Salze

1,1-Methylen-bis-(3-(1-hydroxy-methyl-2,4-dioximidazolidin-5-yl)harnstoff)

Imidazolidinylharnstoff 2-Phenoxy-ethanol Benzylalkohol.

Das erfindungsgemäße kosmetische Mittel kann als Emulsion (Creme oder Milch), als wäßrige oder ölige Lösung, als wäßriges oder öliges Gel, als Stift oder auch als Aerosol vorliegen. Es kann zudem in jeder Form vorliegen, die für kosmetische Hautpflegemittel geeignet oder üblich ist. Die Darstellung erfolgt i.a. durch Mischen und Rühren der Komponenten, gegebenenfalls mit anschließendem Homogenisieren.

Die Erfindung wird im folgenden anhand der Beispiele näher erläutert, ohne daß dies einschränkenden Charakter haben soll.

Beispiel 1

<u>O/W-Emulsion</u> (Feuchtigkeitscreme)     (Angaben in g)

I.   Polyoxyethylen-12-Cetylstearylalkohol      3,0

     Palmitin-/Stearinsäure-Mono/Diglyceride     8,0

     2-Octyldodecanol                           10,0

     Capryl-/Caprinsäuretriglycerid              5,0

     Paraffinöl, dickflüssig                     4,0

II.  Retinalpalmitat                     500.000 I.E.

     alpha-Tocopherol (Vitamin E)               0,1

     3,7,11,15-Tetramethyl-1,2,3-trihydroxy-

               hexadecan                         0,1

III. Parfümöl                                   q.s.

IV.  Glycerin                                    7,0

     Konservierungsmittel                       q.s.

     Wasser                              ad 100,00

Herstellung :

Die Mischung I wird bei 75°C zum Schmelzen gebracht und die auf die gleiche Temperatur erwärmte Lösung IV unter Rühren zugegeben. Unter weiterem Rühren und Homogenisieren läßt man auf 35°C abkühlen, dann gibt man die Mischung II und das Parfümöl (III) hinzu, ergänzt mit Wasser auf 100 g und läßt unter weiterem Rühren auf Raumtemperatur abkühlen.

Die gesamte Herstellung erfolgt unter Vakuum-Bedingungen, um das Einarbeiten von Luft zu vermeiden.

Beispiel 2

<u>O/W-Emulsion</u> (Körpermilch)                    (Angaben in g)

I.    Mono-, Di- u. Tri(alkyltetra-
      glykolether)-o-phosphorsäureester        3,0
      Paraffinöl, dünnflüssig                  3,0
      Isopropylpalmitat                        3,0
      Jojobaöl                                 5,0
      Retinolpalmitat                          500.000 I.E.
      Butylhydroxytoluol                       0,01
      3,7,11,15-Tetramethyl-1,2,3-trihydroxy-
      hexadecan                                0,1
      Parfümöl                                 q.s.

II.   Acrylamid/Na-acrylat-Copolymer           0,6

III.  Glycerin                                 3,0
      Konservierungsmittel                     q.s.

      Wasser                                   ad 100,0

Herstellung :

Die Bestandteile von I werden unter Rühren gemischt. Anschließend wird das Acrylamid/Na-acrylat-Copolymer II mit dem Ultra-Turrax gleichmäßig in der Mischung I suspendiert. Die Lösung III wird unter schnellem Rühren einemulgiert, dann wird die Emulsion homogenisiert.

5

Beispiel 3

W/O-Emulsion (Nachtcreme)                                    (Angabe in g)

| | | |
|---|---|---|
| I. | Polyethylenglycol-1-Glycerol- | |
| | Sorbitan-Isostearat | 10,0 |
| | Bienenwachs | 3,0 |
| | Lanolin | 3,0 |
| | Capryl-/Caprinsäuretriglycerid | 11,0 |
| | Perhydrosqualen | 11,0 |
| | Weizenkeimöl | 3,0 |
| | | |
| II. | Retinolpalmitat | 500.000 I.E. |
| | Butylhydroxyanisol | 0,01 |
| | 3,7,11,15-Tetramethyl-1,2,3-trihydroxy- | |
| | hexadecan | 0,1 |
| | | |
| III. | Parfümöl | q.s. |
| | | |
| IV. | Glycerin | 2,0 |
| | Konservierungsmittel | q.s. |
| | Magnesiumsulfat | 0,7 |
| | | |
| | Wasser | ad 100,0 |

Herstellung :

Die Mischung I wird bei 75°C zum Schmelzen gebracht und die auf die gleiche Temperatur erwärmte Lösung IV unter Rühren zugegeben. Unter weiterem Rühren und Homogenisieren läßt man auf 35°C abkühlen, dann gibt man die Mischung II und das Parfümöl (III) hinzu, ergänzt mit Wasser auf 100 g und läßt unter weiterem Rühren auf Raumtemperatur abkühlen. Die gesamte Herstellung erfolgt in einer evakuierten Apparatur, um das Einarbeiten von Luft zu vermeiden.

Beispiel 4

**W/O-Emulsion** (Hautpflegemilch)          (Angaben in g)

I.   Polyoxypropylen-1,5-polyoxy-
     ethylen-2,5-glycerol-Sorbitan-
     Hydroxystearat                          6,0
     Polyoxypropylen(15)-stearylether/
     Cyclomethicone                          6,0
     Paraffin, dünnflüssig                   12,0

II.  Retinolpalmitat                    500.000 I.E.
     alpha-Tocopherol (Vitamin E)           0,1
     3,7,11,15-Tetramethyl-1,2,3-trihydroxy-
     hexadecan                               0,1

III. Parfümöl                                q.s.

IV.  Polyethylenglycol-Sorbitol             4,0
     Magnesiumsulfat                         0,7
     Konservierungsmittel                    q.s.

     Wasser                              ad 100,0

Die Herstellung der W/O-Emulsion (Hautpflegemittel) erfolgt in Analogie zu den Angaben von Beispiel 1.

WIRKSAMKEITSVERGLEICH

1. Meßverfahren

Für jede Versuchsreihe werden 5 weiblichen Rhinomäusen (Alter 7-9 Wochen) täglich je 0.025 ml der Testlösung topisch appliziert. Die Versuchsdauer beträgt 9 Tage. Danach werden Zellschnitte von Biopsien der behandelten Haut optisch vermessen und ausgewertet.

Zur Ergebnisbewertung werden einerseits der durchschnittliche Utriculumumfang (U), andererseits die durchschnittliche Epidermisdicke herangezogen.

Folgende Versuchsreihen soll die Wirkung der erfindungsgemäßen Rezepturen verdeutlichen :

Versuchsreihe 1 :

```
Gemisch aus      5000 I.E./g Retinolpalmitat
                 0,1 Gew.-%  3,7,11,15-Tetramethyl-
                             1,2,3-trihydroxy-hexadecan
                             (= Phytantriol)
                 0,1 Gew.-%  alpha-Tocopherol ( = Vitamin
                             E) in Laurinsäurehexylester
```

Versuchsbeispiel 2.

```
Gemisch aus      5000 I.E./g Vitamin-A-Palmitat in
                             Laurinsäurehexylester
```

Versuchsreihe 3 : Laurinsäurehexylester als Placebo

Versuchsreihe 4 : ohne Behandlung (O-Kontrolle).

2. Ergebnisse :

| | Durchmesser U (nm) | %-Durchmesser U der Kontrolle | Wirksamkeit (in %) | Epidermis Dicke (nm) | Faktor der Zunahme der Epidermisdicke |
|---|---|---|---|---|---|
| Versuchsreihe 1 | 78 | 36 | 64 | 67 | 4,5 |
| Versuchsreihe 2 | 120 | 55 | 45 | 49 | 3,3 |
| Versuchsreihe 3 | 216 | 99 | 1 | 37 | 2,5 |
| Kontrolle | 218 | 100 | 0 | 15 | 1,0 |

Die erfindungsgemäße Rezeptur verringert somit die Utrikulumdurchmesser im Durchschnitt um 64% und erhöht die Epidermisdicke auf das 4,5 fache des Ausgangswertes, während die gleiche Wirkstoffmenge in neutraler Grundlage (Versuchsreihe 2) wesentlich geringere Verbesserungen und die Placeboreihe 3 nur sehr geringe Effekte aufweist.

**Patentansprüche**

1. Kosmetisches Präparat auf Basis von Retinolpalmitat in flüssiger, halbfester oder fester Form, dadurch

8

gekennzeichnet, daß es neben üblichen Trägern und/oder Hilfsstoffen Retinolpalmitat in einer Menge von 1000 bis 15000 I.E./g und 0,01 bis 1 Gewichtsprozent 3,7,11,15-Tetramethyl-1,2,3-trihydroxyhexadecan enthält.

2. Kosmetisches Präparat gemäß Anspruch 1, dadurch gekennzeichnet, daß es sich um eine Öl-in-Wasser-Emulsion, Wasser-in-Öl-Emulsion, eine wäßrige oder ölige Lösung, ein Gel, einen Stift oder ein Aerosol handelt.

3. Kosmetisches Präparat gemäß Anspruch 1, dadurch gekennzeichnet, daß es 2000 bis 5000 I.E./g Retinolpalmitat und 0,05 bis 0,5 Gew.-% 3,7,11,15-Tetramethyl-1,2,3-trihydroxy-hexadecan enthält.

4. Kosmetisches Präparat gemäß Anspruch 1 bis 3, dadurch gekennzeichnet, daß es als Träger- und/oder Hilfsstoffe einen oder mehreren der folgenden Stoffe enthält : Antioxidantien, Lösungsmittel, mineralische, tierische oder pflanzliche Öle oder Wachse, Fettsäuren, Fettalkohole, Fettsäureester, Fettalkoholether, oxyethylierte Fettalkohole, Lanolin oder Lanolinderivate, Siliconöle, Emulgatoren, Verdikkungsmittel, Feuchthaltemittel, UV-Filter, Farbstoffe, Puffersubstanzen, Konservierungsmittel und Parfümöle.

5. Verfahren zur Herstellung von kosmetischen Präparaten auf Basis von Retinolpalmitat, dadurch gekennzeichnet, daß man pro g des kosmetischen Präparates 1000 bis 15000 I.E. Retinol-palmitat und bezogen auf das kosmetische Präparat 0,01 bis 1 Gew.-% 3,7,11,15-Tetramethyl-1,2,3-trihydroxy-hexadecan mit üblichen Träger- und/oder Hilfsstoffen gegebenenfalls unter Rühren und/oder Homogenisieren vermischt.

## Claims

1. Cosmetic preparation based on retinol palmitate in liquid, semi-solid or solid form, characterised in that, in addition to customary carriers and/or auxiliaries, it contains retinol palmitate in an amount of 1000 to 15000 I.U./g and 0.01 to 1 per cent by weight of 3,7,11,15-tetramethyl-1,2,3-trihydroxy-hexadecane.

2. Cosmetic preparation according to Claim 1, characterised in that it is an oil-in-water emulsion, water-in-oil emulsion, an aqueous or oily solution, a gel, a stick or an aerosol.

3. Cosmetic preparation according to Claim 1, characterised in that it contains 2000 to 5000 I.U./g of retinol palmitate and 0.05 to 0.5% by weight of 3,7,11,15-tetramethyl-1,2,3-trihydroxy-hexadecane.

4. Cosmetic preparation according to Claims 1 to 3, characterised in that, as carriers and/or auxiliaries, it contains one or more of the following substances : antioxidants, solvents, mineral, animal or vegetable oils or waxes, fatty acids, fatty alcohols, fatty acid esters, fatty alcohol ethers, oxyethylated fatty alcohols, lanolin or lanolin derivatives, silicone oils, emulsifiers, thickeners, humectants, UV filters, colorants, buffer substances, preservatives and perfume oils.

5. Method for the production of cosmetic preparations based on retinol palmitate, characterised in that 1000 to 15000 I.U. of retinol palmitate per g of the cosmetic preparation and 0.01 to 1% by weight of 3,7,11,15-tetramethyl-1,2,3-trihydroxy-hexadecane relative to the cosmetic preparation are mixed with customary carriers and/or auxiliaries, optionally with stirring and/or homogenisation.

## Revendications

1. Préparation cosmétique à base de palmitate de rétinol sous forme liquide, semi-solide ou solide, caractérisée en ce qu'elle contient, à côté de supports et/ou de substances auxiliaires classiques, du palmitate de rétinol en une quantité de 1000 à 15 000 U.I./g et 0,01 à 1% en poids de 3,7,11,15-tétraméthyl-1,2,3-trihydroxyhexadécane.

2. Préparation cosmétique suivant la revendication 1, caractérisée en ce qu'elle est une émulsion huile-dans-eau, une émulsion eau-dans-huile, une solution aqueuse ou huileuse, un gel, un bâton ou un aérosol.

3. Préparation cosmétique suivant la revendication 1, caractérisée en ce qu'elle contient 2000 à 5000 U.I./g de palmitate de rétinol et 0,05 à 0,5% en poids de 3,7,11,15-tétraméthyl-1,2,3-trihydroxyhexadécane.

4. Préparation cosmétique suivant les revendications 1 à 3, caractérisée en ce qu'elle contient comme supports et/ou comme substances auxiliaires une ou plusieurs des substances suivantes : antioxydants, solvants, huiles ou cires minérales, animales ou végétales, acides gras, alcools gras, esters d'acides gras, éthers d'alcools gras, alcools gras oxyéthylés, lanoline ou dérivés de lanoline, huiles de silicone, émulsionnants, agents épaississants, agents humectants, filtres contre les rayons ultraviolets, colorants, substances tampons, agents conservateurs et essences parfumées.

5. Procédé de production de préparations cosmétiques à base de palmitate de rétinol, caractérisé en ce qu'on mélange, par gramme de préparation cosmétique, 1000 à 15000 U.I. de palmitate de rétinol et, par rapport à la préparation cosmétique, 0,01 à 1% en poids de 3,7,11,15-tétraméthyl-1,2,3-trihydroxyhexadécane avec des supports et/ou des substances auxiliaires classiques, le cas échéant sous agitation et/ou avec homogé-

néisation.